# EUROPÄISCHE PATENTSCHRIFT

(11) **EP 1 838 448 B1**
(45) Veröffentlichungstag und Bekanntmachung des Hinweises auf die Patenterteilung: **27.08.2008**
(21) Anmeldenummer: 05803036.2
(22) Anmeldetag: 18.11.2005
(51) Int. Cl.: B02C 19/00, G01N 1/28, C12M 3/08, B02C 18/18

(54) **EINWEG-FRAKTIONIERVORRICHTUNG**
ONE-WAY FRACTIONATING DEVICE
DISPOSITIF DE FRACTIONNEMENT A USAGE UNIQUE

(30) Priorität: 21.01.2005 CH 912005
(43) Veröffentlichungstag der Anmeldung: 03.10.2007
(73) Patentinhaber: Medic Tools AG, 6304 Zug (CH)
(72) Erfinder: BUCHER, Franz, Gregor, CH-6304 Zug (CH)
(74) Vertreter: Gachnang, Hans Rudolf
(86) Internationale Anmeldenummer: PCT/CH2005/000684
(87) Internationale Veröffentlichungsnummer: WO 2006/076819

(56) Entgegenhaltungen:
- EP-A- 0 590 219
- WO-A-20/04035191
- GB-A- 2 374 301
- PATENT ABSTRACTS OF JAPAN Bd. 014, Nr. 299 (C-0733), 27. Juni 1990 (1990-06-27) -& JP 02 098355 A (SHINSAKU KAGUCHI), 10. April 1990 (1990-04-10)

## Beschreibung

Gegenstand der Erfindung ist eine Einweg-Fraktioniervorrichtung gemäss Oberbegriff des Patentanspruchs 1.

Mehrfach einsetzbare Fraktionierwerke sind im Laborbetrieb allgemein bekannt und gebräuchlich. Beim Fraktionieren und Fragmentieren von pflanzlichen Stoffen und Gewebematerialien in nicht hermetisch abgeschlossenen Gefässen und mit herkömmlichen, wieder verwendbaren Fraktionierer und Fragmentierern besteht die latente Gefahr einer Cross-Kontamination von einer Fraktioniergut-Charge zur nächsten, zwischen dem Fraktioniergut und dem die Verarbeitung vorzunehmenden Benutzer sowie der Kontamination eines sterilen Fragmentierguts durch das Umfeld.

Aus der WO 2004/035191 ist ein Einweg-Mischer und -homogenisator bekannt, der auf ein Labortestgefäss aufschraubbar ist und einen im Labortestgefäss eingebrachten Stoff mischen und homogenisieren, aber auch durch die Messer und entsprechenden Gegenmesser zerkleinern, d.h. fraktionieren kann. Diese bekannte Vorrichtung eignet sich vorzüglich zum Mischen, wird sie jedoch als Fraktionierer eingesetzt, so ist nicht sichergestellt, dass nach einer vorgegebenen Fraktionierzeit der gesamte Inhalt, insbesondere wenn dieser eine gewisse Menge überschreitet, die gewünschte gleichmässige Körnung aufweist.

Eine Aufgabe der vorliegenden Erfindung ist die Schaffung einer Einweg-Fraktioniervorrichtung, welche es ermöglicht, den Fraktionier- und Fragmentiervorgang nach dem Einbringen des Fraktionierguts hermetisch von der Umgebung abgeschlossen durchzuführen.

Gelöst wird diese Aufgabe durch eine Einweg-Fraktioniervorrichtung gemäss den Merkmalen des Patentanspruchs 1. Besonders vorteilhafte Ausgestaltungen der Erfindung sind in den abhängigen Ansprüchen umschrieben.

Durch die Anordnung des Fraktioniergehäuses an einem ersten Labortestgefäss mit dem unfraktionierten Fraktioniergut und der Anordnung des Fraktionierwerks auf einem zweiten Labortestgefäss, wobei das Fraktionierwerk drehbar im Fraktioniergehäuse gelagert ist, ermöglicht es, das Fraktioniergut im Durchlauf zu fraktionieren, d.h. bereits fraktioniertes Fraktioniergut gelangt nicht ein zweites Mal in die Fraktioniervorrichtung. Auf diese Weise kann die geforderte Körnung erreicht werden, und es ist jederzeit ersichtlich, wann der Fraktioniervorgang abgeschlossen sein wird. In einer besonders vorteilhaften Ausgestaltung kann der Fraktioniergrad eingestellt werden.

Die Erfindung wird nachfolgend anhand eines illustrierten Ausführungsbeispiels näher erläutert. Es zeigen
- Figur 1: einen Axialschnitt durch das Fraktionierwerk und den Verbindungsflansch für das zweite Labortestgefäss,
- Figur 2: eine Aufsicht auf das Fraktionierwerk,
- Figur 3: einen Axialschnitt durch das Gehäuse des Fraktionierwerks und den Verbindungsflansch für das erste Labortestgefäss,
- Figur 4: eine Aufsicht auf das Gehäuse und
- Figur 5: einen Axialschnitt durch die zusammengesetzte Einweg-Fraktioniervorrichtung,
- Figur 6: einen Axialschnitt durch eine weitere Ausführungsform des Gehäuses des Fraktionierwerks,
- Figur 7: einen Axialschnitt durch das Fraktionierwerk und den Verbindungsflansch für das zweite Labortestgefäss gemäss Figur 6.

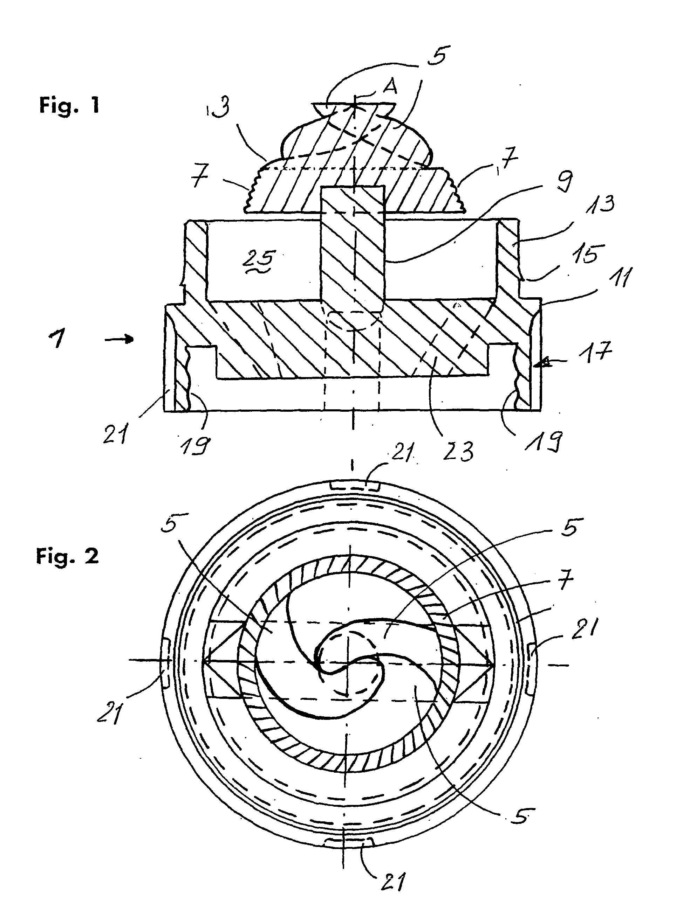

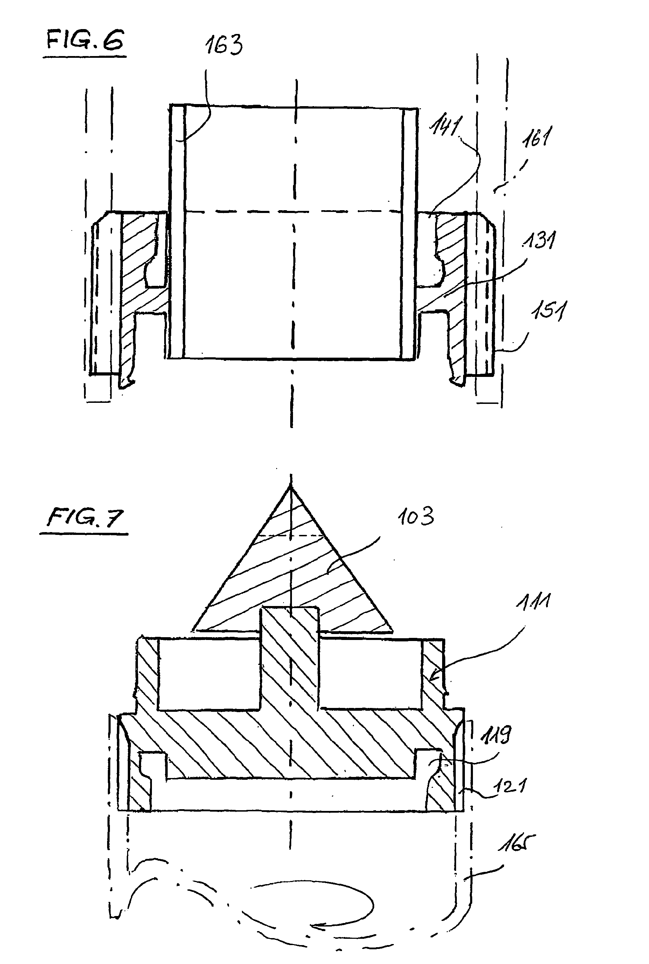

In Figur 1 ist mit Bezugszeichen 1 ein Fraktionierwerk bezeichnet, welches einen Fraktionierkopf 3 mit beispielsweise zwei Zuführschnecken 5 aufweist und mit einem kegelstumpfförmigen Schneidelement 7 ausgestaltet ist. Die einzelnen Schneiden des Schneidelements 7 können, wie aus Figur 2 ersichtlich, schräggestellt sein. Selbstverständlich ist auch eine andere Schneidengeometrie möglich. Der Fraktionierkopf 3 sitzt auf einem Wellenstummel 9, welcher am Fraktionierwerkskörper 11 befestigt ist. Am Fraktionierwerkskörper 11 ist fraktionierkopfseitig ein nach oben gerichteter Kragen 13 mit einer umlaufenden Rückhalterippe 15 ausgebildet. An der Unterseite des Fraktionierwerkskörpers 11 ist ein Flansch 17 mit einem Innengewinde 19 angeformt. Axial verlaufende Nuten 21 sind aussen in die Mantelfläche des Flansches 17 eingelassen. Innerhalb des Kragens 13 sind im Fraktionierwerkskörper 11 diesen durchdringende Ausnehmungen 23 ausgebildet. Diese Ausnehmungen 23 verbinden den zwischen dem Schneidelement 7 und der Oberfläche des Fraktionierwerkskörpers 11 liegenden Raum 25 mit der Unterseite des Fraktionierwerkskörpers 11. Das Innengewinde 19 am Flansch 17 dient zur Befestigung eines Labortestgefässes als Aufnahmebehälter 27 für das fraktionierte Gut 57. Der Aufnahmebehälter 27 weist an seinem oberen Rand ein entsprechend ausgebildetes Aussengewinde 29 auf. Dieses kämmt einerseits am Innengewinde 19 und liegt, wenn vollständig eingedreht, dichtend am Fraktionierwerkskörper 11 an.
Alternativ zu einer Gewindeverbindung zwischen dem Aufnahmebehälter 27 und einem Speisebehälter 43, z.B. Labortestgefässen, und dem Fraktionierwerkskörper 11 bzw. dem Fraktionierwerk 1 kann auch eine Schnappverbindung treten. An die Stelle der Innengewinde 19, 41 treten dann Rillen oder Einstiche 119, 141 (vergl. Figuren 6 und 7) und anstelle der Aussengewinde an den Behältern 27, 43 treten Wulste (keine Abbildungen).

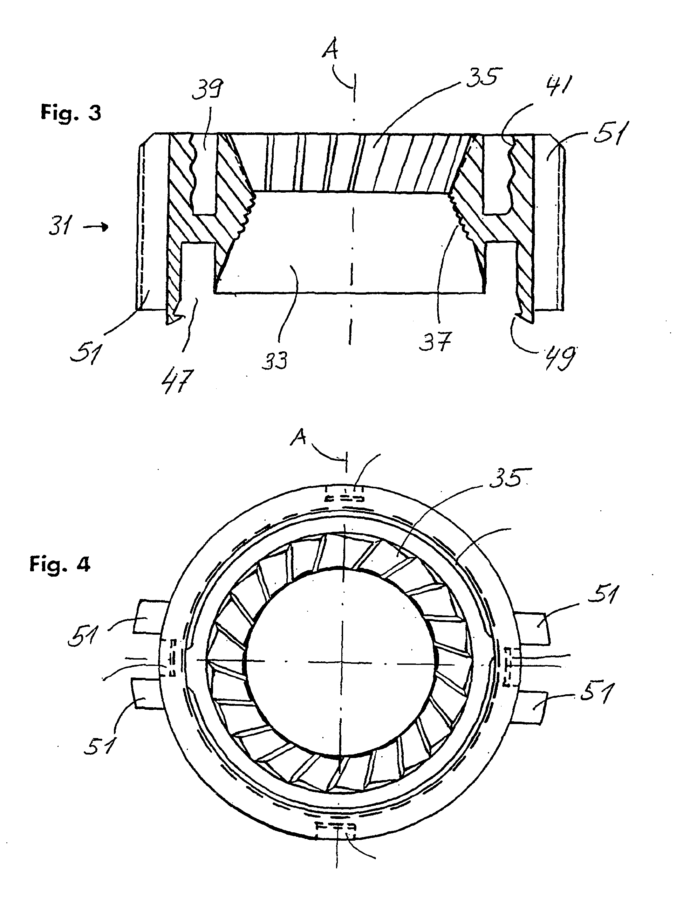

In den Figuren 3 und 4 ist das Gehäuse 31 des Fraktionierwerks 1 dargestellt. Dieses umfasst eine doppel-konische Öffnung 33, an der oben am Umfang eine Vielzahl von Leitelementen 35 und unten Schneidelemente 37 ausgebildet sind. Die Konizität der Öffnung im Bereich der Schneidelemente 37 entspricht etwa derjenigen der Schneidelemente 7 auf dem Fraktionierkopf 3. Ausserhalb der doppelkonischen Öffnung 33 ist von oben im Gehäuse 31 eine kreisringförmige Nut 39 eingelassen, an deren äusseren Flanke ein Innengewinde 41 ausgebildet ist. Dieses kommt in Eingriff mit einem als Speisebehälter fungierenden Labortestgefäss 43 bzw. mit einem an dessen Rand angebrachten Aussengewinde 45. An der Unterseite des Gehäuses 31 ist eine zweite umlaufende Nut 47 eingelassen, an deren äusseren Flanke eine umlaufende Rastrippe 49 angeformt ist. Auf der Peripherie des Gehäuses 31 sind paarweise axial verlaufende Aufnahmerippen 51 ausgebildet.

Die Aufnahmerippen 51, 151 liegen einander achssymmetrisch gegenüber und dienen dazu, in Eingriff mit Haltemitteln 161 zu gelangen, welche eine Drehung des Fraktionierwerkkörpers 31, 131 verhindern (vergl. auch Fig. 6/7).

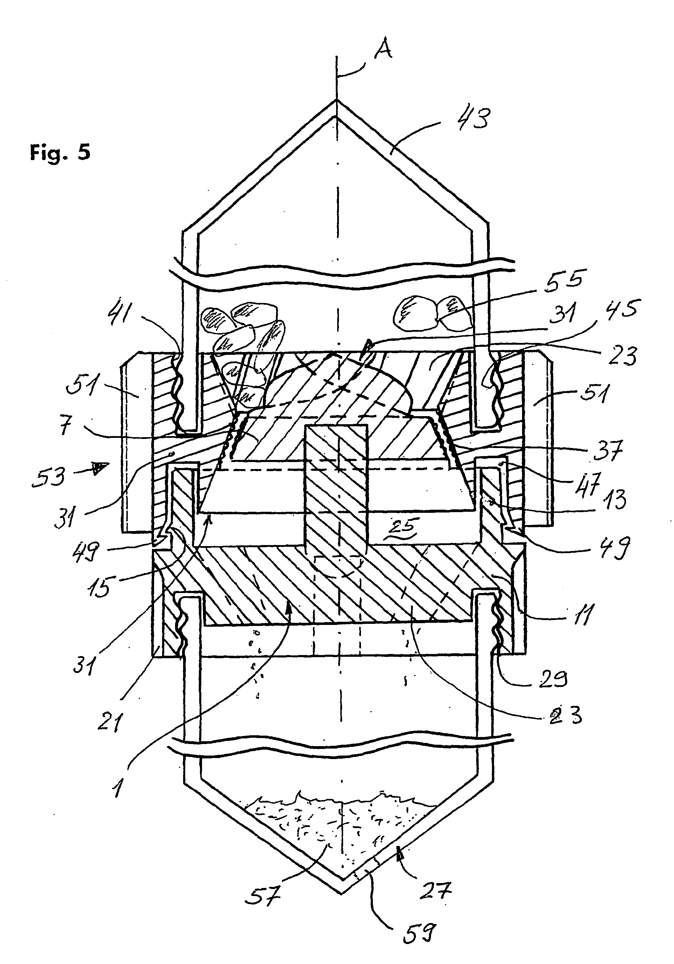

Die Figur 5 zeigt die vollständig zusammengebaute Einweg-Fraktioniervorrichtung 53. Dabei ist ersichtlich, dass auf der Öffnung des Aufnahmebehälters 27 direkt das Fraktionierwerk 1 sitzt und auf der Öffnung des Speisebehälters 43 das Gehäuse 31 für das Fraktionierwerk 1. Das Fraktionierwerk 1 und das Gehäuse 31 werden durch die Rastrippen 15 am Fraktionierwerk und die Rastrippe 49 am Gehäuse 31 zusammengehalten. Die Verbindung dieser beiden Teile ist derart ausgestaltet, dass eine gegenseitige Drehbewegung um die Längsachse A erfolgen kann. D.h. der Kragen 13 am Fraktionierwerk 1 liegt mit Spiel in der Nut 47 am Gehäuse 31.
Im Labortestgefäss 43 sind schematisch die zu fraktionierenden Stoffe mit Bezugszeichen 55 und im Aufnahmebehälter die fraktionierten Stoffe mit Bezugszeichen 57 bezeichnet.

In der weiteren Ausgestaltung der Erfindung gemäss den Figuren 6 und 7 tritt an die Stelle der doppel-konischen Öffnung 33 im Gehäuse 131 eine zylindrische Bohrung in einem Rohr 163 aus Metall oder einem harten Kunststoff.

Die Oberfläche der Bohrung ist entweder gerillt, gerastert, randriert oder auf andere Weise aufgerauht. Der dazugehörige Fraktionierkopf 103 am Fraktionierwerk 101 kann die Gestalt eines Kegels, Kegelstumpfs oder einer Pyramide mit vieleckiger Grundfläche aufweisen. Der Durchmesser der Basisfläche des Kegels oder der Pyramide ist der zu erreichenden Korngrösse entsprechend kleiner als der Innendurchmesser des Rohres 163.

Nachfolgend wird der Fraktioniervorgang beschrieben. In das Labortestgefäss 43 werden an der Teststoff-Entnahmestelle, z.B. auf einem Schiff, an einem Sack oder Silo, unfraktionierte Stoffe 55, z.B. Getreidekörner, eingefüllt. Danach wird das Gehäuse 31, welches mit dem Fraktionierwerk 1 und dem daran befestigten Aufnahmebehälter 37 von oben auf das Labortestgefäss 43 aufgeschraubt und damit hermetisch verschlossen. Vor der Fraktionierung wird die Fraktioniervorrichtung 53 in die in Figur 5 gezeigte Lage gedreht, so dass das Labortestgefäss 43 kopfsteht. Anschliessend wird das Fraktionierwerk 53 im Labor auf einen geeigneten Antrieb 165 aufgesetzt. Dieser greift von unten in die Nuten 21,121 formschlüssig am Fraktionierwerk 1 ein. In die Aufnahmerippen 51,151 greift von oben ein Rastelement 161 ein, welches eine Drehung des Gehäuses 31,131 verhindert. Wenn nun der Antrieb 165 das Fraktionierwerk 1 zusammen mit dem Aufnahmebehälter 27 in Drehung versetzt wird, werden von der Zuführschnecke 5 oder dem Kegel 105 die Stoffe 55 den Schneidelementen 7 und 37 zugeleitet und dort im Durchlauf kontinuierlich fraktioniert. Das Fraktioniergut 57 gelangt in den Raum 25 und von dort durch die Ausnehmungen 23 im Fraktionierwerkskörper 11 in den Aufnahmebehälter 27. Dort kann es nach dem Lösen der Verbindung zwischen dem Aufnahmebehälter 27 und dem Fraktionierwerkskörper 11 entnommen werden.

Während des gesamten Fraktioniervorganges können weder von aussen Verunreinigungen in das Fraktioniergut gelangen noch kann Fraktioniergut aus der Fraktioniervorrichtung 53 nach aussen gelangen. Nicht fraktionierte Stoffe 55 und nicht benutztes Fraktioniergut 57 kann zusammen mit dem Fraktionierwerk nach Gebrauch entsorgt werden.

Selbstverständlich könnte alternativ über die Aufnahmerippen 51 das Gehäuse 31 mit dem Labortestgefäss 43 in Drehung versetzt und das Fraktionierwerk 1 mit dem Aufnahmebehälter drehfest gehalten werden.

Der Fraktionierkopf 3 mit den Schneidelementen 7 kann in einer weiteren Ausgestaltung der Erfindung axial eingestellt werden. Durch die axiale Verschiebung kann der Abstand zwischen den Schneidelementen 7 und den Schneidelementen 37 am Gehäuse 31 ein- und verstellt werden. Dadurch lässt sich die Feinheit, d.h. der Fraktioniergrad, einstellen.

In einer weiteren vorteilhaften Ausgestaltung ist für die Entnahme der fraktionierten Stoffe 57 die Wand des Aufnahmebehälters 27 an einer Stelle mit einem Durchstichbereich 59 versehen, z.B. mit einer Membran, durch welche eine Pipette oder eine andere Entnahmevorrichtung hindurchgeführt werden kann.

Die Fraktioniervorrichtung 53 kann je nach Dimensionierung Speise- bzw. Aufnahmebehälter 27 aufweisen, in denen wenige Kubikzentimeter zu fraktionierende Stoffe 55 Platz finden; es kann aber auch eine Dimension haben, in der beispielsweise ein Kilogramm zu fraktionierende Getreidekörner aufgenommen und fraktioniert werden können. Die gesamte Fraktioniervorrichtung 53 ist kostengünstig aus Kunststoff oder aus kostengünstigen Metallelementen herstellbar.

## Patentansprüche

1. Einweg-Fraktioniervorrichtung (53) zum Fraktionieren oder Fragmentieren von gentechnisch-veränderten, infektiösen, übel riechenden, chemisch aggressiven oder steril zu haltenden Stoffen (55),
**dadurch gekennzeichnet, dass**
das Fraktionierwerk (1) zwischen den Öffnungen zweier axial übereinander liegender Behälter oder Labortestgefässen (27,43) eingesetzt ist, wobei der erste Behälter (27) mit dem Fraktionierkopf (3) und der zweite Behälter (43) mit dem mit dem Fraktionierkopf (3) zusammenwirkenden Fraktionierwerkskörper (11) verbunden ist und dass der Fraktionierkopf (3) mit dem ersten Behälter (27) und der Fraktionierwerkskörper (11) mit dem zweiten Behälter (43) um die Achse (A) relativ zueinander drehbar ausgebildet sind.

2. Einweg-Fraktioniervorrichtung nach nach Anspruch 1, **dadurch gekennzeichnet, dass** der Fraktionierkopf (3) drehfest auf und axial beabstandet zu dem scheibenförmigen Fraktionierwerkskörper (11) angeordnet ist und dass im Fraktionierwerkskörper (11) Ausnehmungen (23) ausgebildet sind, welche eine Verbindung zwischen dem Fraktionierkopf (3) und dem am Fraktionierwerkskörper (11) befestigten Behälter (27) herstellen.

3. Einweg-Fraktioniervorrichtung nach Anspruch 2, **dadurch gekennzeichnet, dass** am Fraktionierwerkskörper (11) eine doppelt-konische Öffnung angebracht ist, in welche von unten der Fraktionierkopf (3) eingreift und die oben mit einer vom Fraktionierkopf (3) getragenen Zuführschnecke (5) zusammenwirkt.

4. Einweg-Fraktioniervorrichtung nach Anspruch 2 oder 3, **dadurch gekennzeichnet, dass** in den Stirnflächen des Fraktionierwerkskörpers (11) oben eine Nut (39) zum Einschrauben oder Einrasten des zweiten Behälters (43) und unten eine Nut (47) zum Einführen und zur Drehlagerung des am Fraktionierwerkskörper (11) angebrachten Kragens (13) angebracht sind.

5. Einweg-Fraktioniervorrichtung nach Anspruch 4, **dadurch gekennzeichnet, dass** auf der Peripherie des Kragens (13) eine erste Rastrippe (15) ausgebildet ist, welche mit einer zweiten Rastrippe (49) in der Aussenflanke der Nut (41) zusammenwirkt und den Mahlwerkskopf (3,103) drehbar mit dem Gehäuse (31,131) verbindet.

6. Einweg-Fraktioniervorrichtung nach einem der Ansprüche 1, 2, 4 oder 5, **dadurch gekennzeichnet, dass** das Gehäuse (131) des Fraktionwerks (101) eine zylindrische Bohrung in einem rohrförmigen Abschnitt aufweist, in welche Bohrung ein kegel-, kegelstumpf-, pyramiden oder pyramidenstumpfförmiger Fragmentierkopf (103) am Fragmentierwerkskörper (111) eingreift.

7. Einweg-Fraktioniervorrichtung nach einem der Ansprüche 1 bis 6, **dadurch gekennzeichnet, dass** am Aufnahmebehälter (27) für das fragmentierte Gut (57) ein durchstechbarer Bereich (59) für die Entnahme von fragmentierten Stoffen ((57) ausgebildet ist.

8. Einweg-Fraktioniervorrichtung nach Anspruch 1, **dadurch gekennzeichnet, dass** die Behälter (27,43) mit Schnappverschlüssen mit dem Fragmentierwerk (1,101) verbunden sind.

## Claims

1. One-way fractionating device (53) for fractionating or fragmenting genetically modified, infectious, evil-smelling, chemically aggressive substances (55) or substances (55) to be kept sterile, **characterised in that** the fractionating mechanism (1) is inserted between the openings of two containers or laboratory test receptacles (27, 43), which are situated axially one above the other, the first container (27) being connected to the fractionating head (3), and the second container (43) being connected to the fractionating mechanism body (11) which co-operates with the fractionating head (3), and **in that** the fractionating head (3) with the first container (27) and the fractionating mechanism body (11) with the second container (43) are configured to be rotatable relative to each other about the axis (A).

2. One-way fractionating device according to claim 1, **characterised in that** the fractionating head (3) is disposed non-rotatably on and axially spaced from the disc-shaped fractionating mechanism body (11), and **in that** recesses (23) are provided in the fractionating mechanism body (11) which establish a connection between the fractionating head (3) and the container (27) secured on the fractionating mechanism body (11).

3. One-way fractionating device according to claim 2, **characterised in that** a biconical opening is provided on the fractionating mechanism body (11), in which opening the fractionating head (3) engages from below, and which opening co-operates at the top with a screw conveyor (5) carried by the fractionating head (3).

4. One-way fractionating device according to claim 2 or 3, **characterised in that**, in the end faces of the fractionating mechanism body (11), a groove (39) is provided at the top for the screwing-in or locking-in of the second container (43), and a groove (47) is provided at the bottom for the introduction and rotatable mounting of the collar (13) provided on the fractionating mechanism body (11).

5. One-way fractionating device according to claim 4, **characterised in that**, on the periphery of the collar (13), a first locking rib (15) is provided, which co-operates with a second locking rib (49) in the outer side of the groove (41) and rotatably connects the grinding mill head (3, 103) to the housing (31, 131).

6. One-way fractionating device according to one of claims 1, 2, 4 or 5, **characterised in that** the housing (131) of the fractionating mechanism (101) has a cylindrical bore in a tubular portion, in which bore engages a conical, frustoconical, pyramidal or truncated pyramidal fragmenting head (103) on the fragmenting mechanism body (111).

7. One-way fractionating device according to one of claims 1 to 6, **characterised in that** a traversable region (59) for the removal of fragmented substances (57) is provided on the receiving container (27) for the fragmented material (57).

8. One-way fractionating device according to claim 1, **characterised in that** the containers (27, 43) are connected to the fragmenting mechanism (1, 101) by means of snap closures.

## Revendications

1. Dispositif ( 53 ) de fractionnement à usage unique pour fractionner ou fragmenter des substances ( 55 ) modifiées génétiquement, infectieuses, malodorantes, agressives chimiquement ou à conserver de manière stérile,
**caractérisé en ce que**
l'outil ( 1 ) de fractionnement est inséré entre les ouvertures de deux récipients ou enceintes ( 27, 43 ) de test de laboratoire superposés axialement, le premier récipient ( 27 ) étant relié à la tête ( 3 ) de fractionnement et le deuxième récipient ( 43 ) à la pièce ( 11 ) de l'outil de fractionnement opérant avec la tête ( 3 ) de fractionnement et **en ce que** la tête ( 3 ) de fractionnement avec le premier récipient ( 27 ) et la pièce ( 11 ) de l'outil de fractionnement avec le deuxième récipient ( 43 ) sont constitués de manière à pouvoir tourner l'une par rapport à l'autre autour de l'axe ( A ).

2. Dispositif de fractionnement à usage unique suivant la revendication 1, **caractérisé en ce que** la tête ( 3 ) de fractionnement est montée fixe en rotation et à distance axialement par rapport à la pièce ( 11 ) de l'outil de fractionnement en forme de disque et **en ce que** dans la pièce ( 11 ) de l'outil de fractionnement sont formés des évidements ( 23 ) qui ménagent une liaison entre la tête ( 3 ) de fractionnement et le récipient ( 27 ) fixé à la pièce ( 11 ) de l'outil de fractionnement.

3. Dispositif de fractionnement à usage unique suivant la revendication 2, **caractérisé en ce que** sur la pièce ( 11 ) de l'outil de fractionnement est ménagée une ouverture doublement conique dans laquelle la tête ( 3 ) de fractionnement pénètre par le bas et qui coopère en haut avec une vis ( 5 ) d'apport portée par la tête ( 3 ) de fractionnement.

4. Dispositif de fractionnement à usage unique suivant la revendication 2 ou 3, **caractérisé en ce que** dans les surfaces frontales de la pièce ( 11 ) de l'outil de fractionnement sont ménagées en haut une gorge ( 39 ) de vissage ou d'encliquetage du deuxième récipient ( 43 ) et en bas une gorge ( 47 ) d'introduction et de montage en rotation du collet ( 13 ) ménagé sur la pièce ( 11 ) de l'outil de fractionnement.

5. Dispositif de fractionnement à usage unique suivant la revendication 4, **caractérisé en ce que** sur la périphérie du collet ( 13 ) est formée une première nervure ( 15 ) d'encliquetage, qui coopère avec une deuxième nervure ( 49 ) d'encliquetage dans le flanc extérieur de la rainure ( 41 ) et qui relie la tête ( 3, 103 ) de l'outil de broyage à rotation avec l'enveloppe ( 31, 131 ).

6. Dispositif de fractionnement à usage unique suivant l'une des revendications 1, 2, 4 ou 5, **caractérisé en ce que** l'enveloppe ( 131 ) de l'outil ( 101 ) de fractionnement a un trou cylindrique dans une partie tubulaire, trou dans lequel pénètre une tête ( 103 ) de fragmentation conique, tronconique, pyramidale ou en forme de tronc de pyramide de la pièce ( 111 ) de l'outil de fragmentation.

7. Dispositif de fractionnement à usage unique suivant l'une des revendications 1 à 6, **caractérisé en ce que** sur le récipient ( 27 ) de réception du produit ( 57 ) fragmenté est formée une partie ( 59 ) pouvant être transpercée pour le prélèvement de substances ( 57 ) fragmentées.

8. Dispositif de fractionnement à usage unique suivant la revendication 1, **caractérisé en ce que** les récipients ( 27, 43 ) sont reliés à l'outil ( 1, 101 ) de fragmentation par des fermetures à déclic.
